# EUROPEAN PATENT SPECIFICATION

(11) **EP 1 940 344 B1**
(45) Date of publication and mention of the grant of the patent: **11.11.2009**
(21) Application number: 06805975.7
(22) Date of filing: 29.09.2006
(51) Int. Cl.: A61K 8/26, A61K 8/28, A61K 8/34, A61Q 15/00

(54) **ANTIPERSPIRANT OR DEODORANT COMPOSITIONS**
SCHWEISSHEMMENDE ODER DESODORIERENDE ZUSAMMENSETZUNGEN
COMPOSITIONS ANTI-TRANSPIRANTES OU DEODORANTES

(30) Priority: 28.10.2005 US 731011 P
(43) Date of publication of application: 09.07.2008
(73) Proprietor: Unilever PLC, London EC4Y 0DY (GB); Unilever N.V., 3013 AL Rotterdam (NL)
(72) Inventor: MASSARO, Michael, Trumbull, Connecticut 06611 (US); MUSCATT, Joseph, Bebington, Wirral Merseyside CH63 3JW (GB); TURNER, Graham, Andrew, Bebington, Wirral Merseyside CH63 3JW (GB)
(74) Representative: Whaley, Christopher
(86) International application number: PCT/EP2006/009502
(87) International publication number: WO 2007/048480

(56) References cited:
- WO-A-98/27947
- WO-A-03/070210
- WO-A2-2005/025523
- WO-A2-2006/001839

## Description

The invention relates to antiperspirant or deodorant compositions, and especially anhydrous compositions.

### Background and prior art

The deodorant and antiperspirant market is dominated by products based on astringent aluminium and/or zirconium salts that are intended to prevent, or at least inhibit, perspiration locally through the skin, particularly in the underarm. By so doing, the user is able to prevent or at least hinder the formation of wet patches under the arm or in clothing that is worn over the arms that some societies consider to be unsightly. By so controlling the volume of sweat on the skin surface, the user simultaneously restricts the supply of organic compounds to the resident population of bacteria on the skin that transform such compounds to malodorous compounds, and thus in that way, the astringent salts act as deodorants as well. Such astringent antiperspirant salts can also act as a bactericide, and thus act as a deodorant, even when applied in an amount less than would be needed to achieve effective antiperspirancy.

However, a side effect of such astringent salts has been observed, namely that they tend to dry the skin and in particular the stratum corneum and it loses to some extent its elasticity. The benefit of overcoming such a disadvantage has been recognised in for example EP966258.

Many variations in form have been suggested for compositions containing an astringent antiperspirant salt intended to be topically applied to skin, for example liquid formulations to accommodate application by spraying, the spray commonly being generated by a pump or by a propellant. Other compositions are intended to be rubbed across the skin, so-called contact formulations, and comprise a carrier medium, in which the antiperspirant salt is dissolved or suspended, that is gelled (solidified, structured) to form a solid or semi-solid formulation. The compositions and particularly contact compositions can be either anhydrous or hydrous, depending on the nature of the composition and any carrier liquid.

In certain variations that are particularly popular in North America, and available elsewhere, contact compositions are anhydrous and comprise a particulate astringent salt suspended in a gelled water-immiscible oil. As with other types of antiperspirant compositions, it is desirable to incorporate a means to counteract drying of contacted skin. It has already been suggested to employ an humectant, and particularly a polyhydric humectant, for such a purpose, and it has also been recognised that the incorporation of such an humectant into such anhydrous compositions can suffer from the problem of grit formation. Although the instant invention is not dependent upon the truth of any particular assumption, postulate, supposition, theory or belief, polyols would seem to be capable of migrating to the surface of particulate antiperspirant salts at temperatures commonly employed to disperse or dissolve the gellant in the carrier oil, and thereafter act as a binder between and thereby agglomerating particles. Grit in a contact composition is particularly undesirable, in that it can be perceived by the user that at best is an unpleasant sensation and at worst can irritate or abrade the skin. Indeed, the problem can be exacerbated by the practice that is common in North America (and some other parts of the world) of users removing hair from the underarm by shaving or plucking before application of the antiperspirant, thereby sensitising the skin. Indeed, skin irritation, whether caused or exacerbated by grit or by any other source is discomforting or painful to users and a disincentive for them to continue to use the product or purchase it again.

Grit formation can also reduce the bio-activity of the formulation, by which is meant the capability of an active ingredient to perform a desired function, such as skin moisturisation by a polyol. Again without being bound by theory, bioactivity of a polyol is impaired if or when the polyol is complexed with the antiperspirant active, unless or until released, so that complexing may contribute to such bio-activity reduction.

It has hitherto been suggested that the problem of grit formation can be counteracted in two ways. In one way, described in more detail in EP966258, an additional material, a perfume carrier, is mixed with the humectant, and especially with glycerol, prior to coming into mixture with the antiperspirant salt. Whilst this way can and has been employed effectively since early 1997, it imposes various constraints on the manufacturer. For example, it circumscribes to at least some extent his formulation options. Any additional material incorporated in a composition, to solve the gritting problem denies formulation space for other ingredients. Moreover, incorporation of a' perfume carrier risks delaying or could hinder the release of perfume from the composition on topical application, thereby reducing its impact and at least the immediate bio-availability of any material absorbed in the carrier.

A second way of at least ameliorating grit formation from an antiperspirant salt and an humectant during manufacture of an anhydrous antiperspirant stick composition has been proposed by Reheis Inc. in USP6649153. Reheis proposes to complex the humectant and the antiperspirant salt during manufacture of the salt. A similar concept is contemplated in WO 03/070210 to Unilever et al. In earlier times, before the problem of gritting had been mentioned, complexes of antiperspirant salts and a polyol were disclosed or mentioned in US 3981986, EP217012, GB1267959, GB1159685, ES3873686, US4089120 and US3792070. Although complexing the polyol with the antiperspirant salt can obviate the problem of gritting during manufacture of antiperspirant compositions, the polyol must be released from the complex before it can accomplish skin moisturisation, so that it is not immediately available for moisturisation when the composition is applied. Polyols readily complex with astringent aluminium and zirconium salts, so that de-complexing is not a rapid or easy process in situ on skin. In consequence, the effectiveness of the complexed polyol as a moisturiser of skin is significantly inhibited, or even not detectable.

WO 01/70185 discloses antiperspirant compositions structured with dibenzylidene sorbital acetate containing dipropylene glycol for example to assist in solubilising the structurant. The text contemplates the possibility of alternatively employing a polyethylene glycol of molecular weight from 200 to 8000 or methoxypolyethylene glycol from 350 to 5000.

In USP 4280994, antiperspirant compositions are disclosed which contain polyethylene glycol having an average molecular weight of from 950 to 1600, such as from 950 to 1050 or 1300 to 1600 that are stated to be aesthetically and cosmetically more appealing, in comparison with a stick which contained a higher proportion of antiperspirant active, but lacked 6% of a non-ionic surfactant. The aesthetic differences cannot be attributed unambiguously to the intermediate molecular weight polyethylene glycol. When such intermediate molecular weight polyethylene glycols were tested, skin was de-moisturised, the retained moisture in skin being lower at the end of the test than at the beginning.

In US patent application no 2004/0022750, neither contemplating.nor addressing the problem of grit formation, there is described a method of reducing the particle size of antiperspirant actives to not exceeding an average particle size of 2 µm by grinding them in suspension in a non-aqueous liquid vehicle in which the solid antiperspirant active is insoluble. The text exemplifies the use of cyclomethicones (volatile silicones) as the non-aqueous liquid in a weight ratio of liquid to solid of 3:1, but contemplates as the liquid vehicle many other classes of liquids, namely, cosmetic esters, glycols and polyols, non-volatile silicones, hydrocarbons alcohols and mixtures of the foregoing.

### Object of the present invention

It is an object of the present invention to overcome or ameliorate one or more of the problems or disadvantages disclosed hereinabove.

It is an object of certain embodiments of the present invention to devise anhydrous antiperspirant compositions exhibiting positive humectant bio-availability and freedom from sensed grit.

It is an object of various preferred embodiments according to the present invention to provide compositions containing a particulate astringent antiperspirant that counteract irritancy.

### Statement of Invention

According to a first aspect of the present invention, there is provided an anhydrous antiperspirant or deodorant composition comprising
a particulate astringent antiperspirant salt;
a carrier oil,
a gellant, for the oil and
a polyol humectant,
in which the humectant comprises a low molecular weight polyethylene glycol
and in accordance with claim 1 hereinafter.

The polyethylene glycol being liquid and not pre-complexed with the antiperspirant astringent salt can be considered to be "free" polyethylene glycol .

According to a second aspect of the present invention, there is provided a process for ameliorating or preventing grit formation during manufacture of an anhydrous antiperspirant or deodorant composition in accordance with the first aspect comprising the steps of:-
forming at an elevated temperature a fluid mixture comprising
   a particulate astringent aluminium and/or zirconium salt suspended in a carrier oil in which a gellant is dispersed or dissolved therein and
   a polyol humectant
and thereafter cooling or permitting the mixture to cool to a temperature at which the mixture sets in which the humectant comprises a low molecular weight polyethylene glycol.

The resultant composition continues to comprise "free" polyethylene glycol.

According to a third aspect of the present invention, there is provided a non-therapeutic method for the inhibition of perspiration whilst simultaneously ameliorating skin drying by topical application of a composition according to the first aspect.

By employing a low molecular weight polyethylene glycol (sometimes abbreviated herein to PEG) as the humectant, that is not prior complexed with the antiperspirant active, it is possible to form an anhydrous composition containing a significant amount of a polyol humectant that is available immediately to act as a moisturiser upon skin contact without causing gritting, or at least significantly reducing the proportion of grit formed by comparison with incorporation of the same weight proportion of glycerol.

Anhydrous herein means that no separate aqueous liquid phase is present, and not more than 5%, preferably not more than 3%, especially not more than 1% and particularly not more than 0.5% by weight, based on the entire composition of free water. Bound or complexed water, as for example water of hydration in the antiperspirant salt is deemed not to be free.

### Detailed Description of the Invention and Preferred Embodiments thereof.

The present invention relates to the selection of a low molecular weight PEG to provide an instantly available humectant when an antiperspirant or deodorant composition containing an astringent antiperspirant salt is applied to skin which is less susceptible to causing grit formation during composition manufacture at elevated temperatures employed to disperse or dissolve the gellant in the carrier oil. Positive skin hydration can be achieved. It will be recognised that the PEG is included in the composition separate from, i.e. not complexed with, the antiperspirant active.

The invention compositions contemplated herein are in the form of solids or soft solids the latter sometimes being called semi-solids or anhydrous creams. Solids are characterised by retaining their shape without lateral support under the influence of the Earth's gravity. The invention solids are commonly employed in the form of sticks. The hardness of such solids in general and sticks in particular can be measured in a needle penetration test, for example using a lab plant PNT penetrometer equipped with.a Seta wax needle (weight 2.5 grams in a holder of 47.5 grams) which has a cone angle at the point of the needle specified to be 9°10' ± 15", resting on the surface of a flat topped sample, and measuring the depth of penetration after five seconds. Desirably, the depth of penetration is not more than 30mm, and preferably not more than 25mm. Many suitable solids have a penetration of at least 5mm, such as up to 20mm. Semi solids, as their name suggests, are not as hard as solids. They can be extruded through a narrow aperture under the influence of a pressure of around 3psi (about 20. 7kPa) and need a retaining lateral wall to prevent them from slowly spreading. Their hardness when measured by a sphere indentation method is usually higher than 0.005 N/mm², normally below than 0.5 N/mm², and in many compositions the hardness is from 0.01 up to 0.1N/mm². Solids (firm sticks). are indicated by a sphere indentation of higher than 0.5 N/mm².

### Astringent Antiperspirant Salts

The weight proportion of the astringent antiperspirant salt, in the composition or mixture if'more than one salt is employed, is varied at the discretion of the manufacturer and normally in the range of from 0.1 to 60 % by weight of the composition. For employment as a deodorant, the proportion is normally up to 5% by weight often at least 0.5% or at least 1%, and particularly at least 2 or 3%. The effectiveness of the astringent salt to inhibit perspiration increases with increasing weight, so that the proportion is commonly selected in the range of from 5 to 30%, and in many desirable compositions from 10 or 15% up to 26% or 30% by weight.

Astringent antiperspirant salts for use herein are often selected from astringent aluminium, zirconium and mixed aluminium/zirconium salts, optionally complexed. Preferred aluminium, zirconium and aluminium/zirconium salts contain a halide, especially chloride and especially preferred salts are basic salts, which is to say a fraction of the halide within the empirical formula has been replaced by bound hydroxyl groups. Chlorohydrate salts are very highly desired.

Aluminium halohydrates are usually defined by the general formula Al₂(OH)ₓQ_{y}.wH₂0 in which Q represents chlorine, bromine or iodine, x is variable from 2 to 5 and x + y = 6 while wH₂O represents a variable amount of hydration. Aluminium chlorohydrate as made comprises a mixture of a number of different polymeric species in varying proportions, depending on the molar ratio of aluminium to chloride and the conditions employed during manufacture. All such mixtures are employable herein. It is especially desirable to employ what is commonly called activated aluminium chlorohydrate or enhanced activity aluminium chlorohydrate, sometimes abbreviated to AACH, in which the proportion of the more active species, such as Band III species (by a conventional chromatographic method) is higher by virtue of its method of manufacture. In one definition of activated, given in EP 6739, the material has greater than 20% Band III. Other methods of making AACH are given in EP 191628 and EP 451395. AACH is often made by recovery of an aluminium chlorohydrate from a dilute solution under strictly controlled reaction/maturing/dewatering/drying conditions. AACH is commercially available by name, or as activated or enhanced activity, from suppliers such as Reheis, Summit Research and B K Giulini.

Zirconium actives can usually be represented by the empirical general formula : ZrO(OH)_{2n-nz}B₂.wH₂0 in which z is a variable in the range of from 0.9 to 2.0 so that the value 2n-nz is zero or positive, n is the valency of B, and B is selected from the group consisting of chloride, other halide, sulphamate, sulphate and mixtures thereof. Possible hydration to a variable extent is represented by wH₂0. Preferable is that B represents chloride and the variable z lies in the range from 1.5 to 1.87. In practice, such zirconium salts are usually not employed by themselves, but as a component of a combined aluminium and zirconium-based antiperspirant.

The above aluminium and zirconium salts may have coordinated and/or bound water in various quantities and/or may be present as polymeric species, mixtures or complexes. In particular, zirconium hydroxy salts often represent a range of salts having various amounts of the hydroxy group. Zirconium aluminium chlorohydrate may be particularly preferred.

Antiperspirant complexes based on the above-mentioned astringent aluminium and/or zirconium salts can be employed. The complex often employs a compound with a carboxylate group, and advantageously this is an amino acid. Examples of suitable amino acids include dl-tryptophan, d1-(β-phenylalanine, dl-valine, dl-methionine and β-alanine, and preferably glycine which has the formula CH₂(NH₂) COOH.

It is highly desirable in some embodiments of the instant invention to employ complexes of a combination of aluminium halohydrates (especially chlorohydrates) and zirconium chlorohydrates together with amino acids such as glycine, which are disclosed in US-A-3792068 (Luedders et al). Certain of those Al/Zr complexes are commonly called ZAG in the literature. ZAG actives generally contain aluminium, zirconium and chloride with an Al/Zr ratio in a range from 2 to 10, especially 2 to 6, an A1/C1 ratio from 2.1 to 0.9 and a variable amount of glycine. Actives of this preferred type are available from Westwood, from Summit and from Reheis.

It is particularly preferred for the antiperspirant salts to be at least substantially from aluminium sulphate, by which is meant that its weight proportion of the total weight of all antiperspirant salts present is less than 5%, especially less than 3% and particularly less than 1%. Total absence would be very suitable.

Other actives which may be utilised include astringent titanium salts, for example those described in GB 2299506A.

The particle size of the astringent antiperspirant salts feedstock often falls within the range of 0.1 to 100µm and particularly from at least 0.2µm. In many desirable products, the feedstock has at least 95%.by weight of below 50µm with a mean particle size often from 3 to 30µm and in many instances from 5 to 25µm, and in certain highly desirable feedstocks from 10 to 25µm. Advantageously, by selection of the primary polyol humectant in accordance with the present invention, and particularly in preferred embodiments thereof, it is possible to constrain the particle size of the antiperspirant salt in the invention compositions after manufacture at least substantially to below 100 microns, such as at least 95%, preferably at least 99% and particularly 100% of the particles to below 100 microns diameter.

Where it is desired to form antiperspirant products which exhibit no greater than low visible deposits on topical application to skin, it is preferable to select feed-stocks which comprise predominantly non-hollow solid particles, for example not more than 5% or particularly than 2%, especially less than 1% of hollow spherical particles with diameter above 50µm. Hollow particles can be removed by use of suitable grinding apparatus and conditions.

The weight of particulate active antiperspirant salt herein commonly includes any water of hydration present.

### Carrier Oils

The compositions according to the present invention herein comprise at least one carrier oil, by which is meant a compound that is water-immiscible (alternatively describable as hydrophobic or lipophilic) and is liquid at a temperature of 20°C up to at least the temperature at which the gellant is dissolved or dispersed therein and in which the particulate ingredients, such as in particular the antiperspirant salt is suspended. It will be recognised that such dissolution temperature depends mutually on the gellant or mixture of gellants and the oil or mixture of oils. Normally, the oil will have a boiling point in excess of 150°C, and often at least 200°C. The term "carrier oils" herein does not comprise the liquid PEG humectant.

The weight proportion of carrier oils of the invention compositions is commonly selected in the range of from 20 to 90%, and in many instances is at least 30%.

The carrier oils commonly constitute at least 40% by weight of the anhydrous suspension composition, and in many instances at least 45%. The maximum proportion of carrier oils in a stick or soft solid is normally no higher than 90% by weight, in many desirable compositions up to 80% and some particularly preferred compositions is up to 70% w/w of the final composition. Compositions containing from 45 to 60% or 65% carrier oils allow formulation space to readily incorporate an effective amount of antiperspirant salt, for example from 15 to 26% or 30%, moisturiser and enough gellant to achieve a desired hardness. The proportion of carrier oils in the composition is additional to the proportion of the humectant.

Oils employable herein commonly fall into two categories, namely silicone oils (sometimes called organo-silicone oils by virtue of organo-substitution) and non-silicone oils. Also, each of the categories can be divided into two types, namely volatile and non-volatile. Selection of the balance between silicone and non-silicone oils, and between volatile and non-volatile oils is at the discretion of the producer of the cosmetic formulation, who would take into account, amongst other things, the sensory and other physical properties that he wished the resultant product to demonstrate and any constraints arising from choice of gellant (structurant) or additional ingredients.

By volatile herein is meant having a measurable vapour pressure at 25°C. Typically the vapour pressure of a volatile oil lies in a range of at least 1 Pa or preferably at least 10 Pa at 25°C, though generally will be less than 4 kPa (30 mmHg). A non-volatile oil can be considered to generate a vapour pressure of below 1 Pa at 25°C. By his selection of silicone and/or non-silicone oils in varying proportions and volatile and non-volatile oils in varying proportions, compositions having different sensory properties can be obtained.

It is desirable to include volatile silicone because it gives a "drier" feel to the applied film after the composition is applied to skin.

Volatile polyorganosiloxanes can be linear or cyclic or mixtures thereof. Preferred cyclic siloxanes include polydimethylsiloxanes and particularly those containing from 3 to 9 silicon atoms and preferably not more than 7 silicon atoms and most preferably from 4 to 6 silicon atoms, otherwise often referred to as cyclomethicones. Preferred linear siloxanes include polydimethylsiloxanes containing from 3 to 9 silicon atoms. The volatile siloxanes normally by themselves exhibit viscosities of below 10⁻⁵ m²/sec (10 centistokes), and particularly above 10⁻⁷ m²/sec (0.1 centistokes), the linear siloxanes normally exhibiting a viscosity of below 5 x 10⁻⁶ m²/sec (5 centistokes). The volatile silicones can also comprise branched linear or cyclic siloxanes such as the aforementioned linear or cyclic siloxanes substituted by one or more pendant -0-Si(CH₃)₃ groups. Examples of commercially available volatile silicone oils include oils having grade designations 344, 345, ,244, 245 and 246 from Dow Corning Corporation; Silicone 7207^{™} and Silicone 7158™ from Union Carbide Corporation; and SF1202™ from General Electric.

Often, the weight proportion of the volatile silicone oils is at least 10 or at least 20% of the total weight of silicone oils in the composition according to the present invention, and in many particularly suitable compositions, constitutes at least 70% and especially at least 85% by weight of the silicone oils. In other highly desirable compositions according to the present invention, for example when seeking translucent compositions or ones achieving low visible residues, the weight proportion of volatile silicone oils is commonly less than 50%, preferably less than 30%, such as from 0 or 5% to 15 or 20% of the silicone oils.

The carrier oils employed in compositions herein can alternatively or additionally comprise one or more non-volatile silicone oils, which include polyalkyl siloxanes, polyalkylaryl siloxanes and polyethersiloxane copolymers. These can suitably be selected from dimethicone and dimethicone co-polyols. Commercially available non-volatile silicone oils include products available under the trademarks Dow Corning 556 and Dow Corning 200 series. Other non volatile silicone oils include that bearing the trademark DC704. Incorporation of at least some non-volatile silicone oil having a high refractive index such as of above 1.5, eg at least 10% by weight (preferably at least 25% to 100% and particularly from 40 to 80%) of the silicone oils can be beneficial in some compositions, such as where for example it is desirable to reduce visible deposits and/or produce a translucent composition by refractive index matching the dispersed particulate antiperspirant salt with the carrier oil (taking into account the influence of any humectant that forms-a unitary phase with the carrier oil). Many non-silicone oils act as emollients. Any non-silicone oil provides the balance of the silicone oils.

The liquid silicone oils can constitute up to 100% by weight of the water-immiscible liquid carrier oils, for example in many desirable embodiments, their weight proportion is selected in the range of at least 20 or 30% of the carrier oils, often in the range of at least 50% and in some especially preferred embodiments is selected in the range of at least 70% by weight. In various of the above and in other desirable embodiments according to the present invention, non-silicone oils constitute a large or major weight proportion, or even 100% of the oil phase, for example at least 20 or 30%, particularly selected in the range of at least 50% and especially selected in the range of at least 70%.

### Non-silicone oils

The formulator of compositions according to the present invention can include one or more non-silicone oils, sometimes alternatively described as silicon-free hydrophobic or water-immiscible liquids, in addition to or instead of all or a fraction of the silicone oils mentioned hereinbefore. Such oils are, as indicated hereinbefore, liquid at 20°C at standard pressure, indeed are preferably liquid at 15°C and oils having a boiling point of at least 150°C are advantageous. The melting and boiling point data for chemical compounds is readily available in reference works such as the CRC Handbook of Chemistry and Physics published by CRC Press, often together with an indication of whether the compound is water soluble or miscible. For any compound where such data is not available in the literature, it can be measured simply by any chemist using conventional techniques. Various non-silicone oils are volatile and many are non-volatile.

The non-volatile oils, when employed, are often selected from one or more of the following classes of organic compounds, namely, hydrocarbon oils, ester oils and ether oils.

Both volatile and non-volatile hydrocarbon oils are readily available. Volatile oils include, in particular, paraffins and isoparaffins containing an intermediate number of carbon atoms, for example chosen in the range of from 8 to 25 carbons, and often at least 10 carbons, depending on its molecular structure. However, non-ideal mixtures of hydrocarbons tend to have a higher volatility than would be suggested by the individual constituents, and melting and boiling points tend to increase with increasing molecular weight, so such numerical limits represent a guide and indeed there is a diffuse transition to when hydrocarbons are clearly non-volatile. Volatile hydrocarbons can be employed instead of all or a proportion of the volatile silicone oils identified herein before. In many desirable invention formulations, the volatile hydrocarbon comprise from 0 to 20% by weight and especially from 0 to 10% by weight of the total oil blend.

Non-volatile aliphatic hydrocarbons are commonly selected from mineral oils, hydrogenated polydecene and hydrogenated polyisobutene. Non-volatile hydrocarbons can be incorporated to advantage on account of their desirable properties, since many, for example, exhibit emollient properties, the same or others have a low viscosity and by virtue of a mid-range refractive index, such as around 1.46 or 1.47, they generally assist in reducing the visibility of astringent antiperspirant salts when topically adhering to skin or clothing. Non-volatile hydrocarbon oils preferably are present in a proportion of from 0 to 50% w/w, in a number of advantageous embodiments from 0 to 10% w/w of the oils and in other advantageous embodiments of from 10 to 25% w/w of the oils. Suitable non-volatile hydrocarbons include hydrogenated polydecene and petrolatum, the latter commonly being a low melting point waxy material, such as in the region of 35 to 45°C.

Ester oils represent a particularly useful class of non-silicone oils. Other suitable hydrophobic carriers comprise liquid aliphatic or aromatic esters. Typically such oils are regarded as non-volatile. The ester oils can be aliphatic, aromatic or contain both an aliphatic and an aromatic group. Many desirable aliphatic esters contain at least one long chain hydrocarbon group, for example from 8 to 25 carbons, derived from a monohydric alcohol or mono-carboxylic acid. Suitable aliphatic esters can be derived from monohydric alcohols such as selected from C₁ to C₂₀ alkanols esterified with a carboxylic acid selected from C₈ to C₂₂ mono alkanoic acid and C₆ to C₁₀ alkanedioic acids. Such esters include isopropyl myristate, lauryl myristate, isopropyl palmitate, diisopropyl sebacate and diisopropyl adipate. Other suitable ester oils include glyceride oils and in particular triglyceride oils derived from glycerol and fatty acids, sometimes olefinically unsaturated rather than saturated, containing at least 6 carbons and especially natural oils derived from unsaturated carboxylic acids containing from 16 to 20 and especially 18 carbons.

Suitable liquid aromatic esters or mixed aromatic/aliphatic esters are preferably derived from benzoic acid. Examples of such esters include suitable C₈ to C₁₈ alkyl benzoates or mixtures thereof, including in particular C₁₂ to C₁₅ alkyl benzoates. Many suitable benzoate esters are available under the trademark Finsolv. Other aromatic esters which can be contemplated for use herein comprise double aromatic inclusion. Benzyl benzoate, though feasible, is preferably substantially absent, such as at no more than 5%, and particularly no more than 3% or 1% by weight of the oil blend, and more particularly is excluded. Preferred double aromatic esters comprise a linear or branched alkyl chain, e.g. from 1 to 3 carbons, interposed between ester and/or ether substituted phenyl groups.

aliphatic esters tend to exhibit an intermediate refractive index, and are therefore employed typically for their emollient properties. Aromatic esters tend to demonstrate a higher refractive index, such as around 1.49 to 1.50 and when double aromatic substitution is present, even an higher refractive index, rendering them particularly suitable for the preparation of translucent compositions containing a particulate astringent antiperspirant salt, and even salts containing zirconium.

Ester oils, be they aliphatic or aromatic desirably comprise from 0 to 60%, preferably from at least 10 or 15% up to 35 or 40% w/w of the oils, such as highly desirably 15 to 35% in various embodiments. It will recognised that the ester oils mentioned herein are commonly regarded as non-volatile and accordingly can be substituted for non-volatile silicone oils, for example silicone oils of similar refractive index, in whole or in part, at the discretion of the formulator.

In a number of highly desirable embodiments, the invention compositions contain a natural ester oil, either together with or absent any other ester oil. Such natural oils most desirable are glycerides derived from one or more unsaturated C18 fatty acids. In many instances, the oils comprise one or more triglycerides. The fatty acid residues in the oils can comprise, commonly, from one to three olefinic unsaturated bonds and often one or two. Whilst in many instances the olefinic bonds adopt the trans configuration, in a number of desirable products the bond or bonds adopt the cis configuration. If two or three olefinic unsaturated bonds are present, they can be conjugated. The fatty acid can also be substituted by an hydroxyl group. The natural oils employable herein desirably comprise one or more triglycerides of oleic acid, linoleic acid, linolenic acid or ricinoleic acid. Various isomers of such acids often have common names, including linolenelaidic acid, trans 7-octadecenoic acid, parinaric acid, pinolenic acid punicic acid, petroselenic acid and stearidonic acid. It is especially desirable to employ glycerides derived from oleic acid, linoleic acid or petroselenic acid, or a mixture containing one or more of them.

Natural oils containing one or more of such triglycerides include coriander seed oil for derivatives of petroselinic acid, impatiens balsimina seed oil, parinarium laurinarium kernel fat or sabastiana brasilinensis seed oil for derivatives of cis-parinaric acid, dehydrated castor seed oil, for derivatives of conjugated linoleic acids, borage seed oil and evening primrose oil for derivatives of linoleic and linolenic acids, aquilegia vulgaris oil for columbinic acid and sunflower oil, olive oil or safflower oil for derivatives of oleic acid, often together with linoleic acids. Other suitable oils are obtainable from hemp, which can be processed to derive stearadonic acid derivatives and maize corn oil. An especially convenient natural oil by virtue of its characteristics and availability comprises sunflower oil, ranging from those rich in oleic acid glycerides to those rich in linoleic acid glycerides, rich indicating that its content is higher than that of the other named acid.

The proportion of the natural oil in the composition is often selected in the range of from 0.1 to 10% by weight of the carrier mixture, especially in the range of from at least 0.25% by weight and particularly at least 0.5%. Often, its weight proportion is selected in the range of up to 6% by weight and in many embodiments up to 4% of the carrier oils. A particularly convenient range comprises from 0.75 to 3% w/w of the carrier oils.

Ether oils represent further instances of suitable oils. Preferably, the ether oils contemplatable herein comprise liquid aliphatic ethers can be derived from a polyglycol, especially from polypropylene glycol, PPG, the latter preferably containing at least 3 mers, such as 3 to 20, with a monohydric alcohol. The monohydric alcohol often contains between 3 and 20 carbons. As the molecular weight of the PPG increases, so the chain length of the monohydric alcohol can decrease. Hence, for example, suitable ether oils can vary between a low molecular weight PPG with a long chain fatty alcohol, such as PPG-3 myristyl ether and a lower alkyl ethers of a higher molecular weight PPG, such as the ether named as PPG-14 butyl ether in the CTFA Handbook. Such ethers desirably constitute a weight proportion of from 0 to 30%, and preferably at least 5% of the oils, such as conveniently up to 20 or 15%.

In many desirable embodiments according to the present invention, the composition contains at least one ester oil and at least one ether, such as in a weight ratio of total ester: ether of from 5:1 to 1:5. In such or other embodiments, the composition desirably contains both a natural oil and an ether oil, for example selected in a range of weight ratios of from 1:5 to 1:20, and particularly from 1:9 to 1 : 15.

A further class of carrier oils, that can be contemplated herein and which is especially desirable in embodiments employing an amide gellant, comprises water-immiscible aliphatic alcohols that have a boiling point of above 100°C, including in particular branched chain aliphatic alcohols containing from 12 to 25 carbon atoms such as iso-stearyl alcohol and octyldocecanol. In such embodiments, such alcohol oils advantageously contribute from 10 to 50% by weight of the oils.

### Humectant

In the instant invention, the humectant essentially comprises the selection of a low molecular weight polyethylene glycol (PEG) for incorporation in anhydrous suspensions of a particulate antiperspirant salt. Such a material is liquid at ambient temperature or melts at or lower than the processing temperatures commonly employed for making wax-gelled sticks.

The selection is based on identification by the inventors that such humectants meet simultaneously two criteria. First, they can exhibit moisturisation to an extent that is superior for example to propylene glycol and likewise superior to glycols containing 4 to 6 carbon atoms and secondly they can avoid or at least mitigate the risk of grit formation that can arise when glycerol, an excellent humectant, or propylene glycol, an inferior humectant, its employed. A single oligomer or a mixture of polyethylene glycol oligomers can be used. They exhibit good and the preferred exhibit excellent bio-availability, and advantageously exhibit availability during the conventional leave-on period for the compositions on the skin, and generally immediate bio-availability.

The PEG in the instant invention compositions has a low molecular weight, an average molecular weight of not greater than 820, preferably not greater than 620, particularly up to 520 and especially up to 420. The PEG polymer desirable has an average weight of not less than 150 and, in many desirable embodiments, the molecular weight average of the PEG blend is at least 190. PEG polymers of a lower molecular weight tend to be progressively more effective at avoiding antiperspirant active agglomeration, at least until the molecular weight is around 500 or lower and conversely its effectiveness (that is to say as a moisturiser) tends to diminish on a weight/weight basis as its molecular weight increases over 500. This is particularly noticeable at molecular weights of around 1000 or higher.

It is understood that commercially available low molecular weight PEG polymers often comprise a mixture of oligomers. It is especially desirable to employ a polymer (blend) which is free or at least substantially free from the dimer. By substantially free from dimer is meant advantageously less than 5% by weight of the blend, more desirably less than 3% and particularly less than 1%.

Desirably, the total weight proportion of PEG oligomers of formula H- (OCH₂-CH₂)ₙ-OH in which n ≥ 14 units is less than 10% of the weight of PEG, and preferably less than 4%. Advantageously, in at least 95% by weight of the PEG oligomers, n = 3 to 10.

Advantageously, by the selection of a low molecular weight PEG and particularly a preferred PEG, the producer can produce an anhydrous suspension antiperspirant stick or soft stick product having perceivable moisturisation without grit or at the worst, a reduced level of grit, without the need to incorporate at least a predetermined ratio of perfume carrier to humectant. Moreover, the humectant is more readily available to provide skin moisturisation when simply blended into the composition rather than if it were prior complexed with the antiperspirant active.

Although it may be convenient to incorporate a low proportion of PEG polymer in the composition, such as 0:1%, it is preferable to employ a higher proportion and advantageously at least 0.5% therein, in order to increase the moisturisation effect. It is preferable to incorporate at least 1% and many attractive compositions contain at least 1.5% by weight thereof. Its proportion is attractively not more than 10% and in many desirable embodiments is up to 7.5%, and particularly up to 5% by weight of the composition. It is preferred to scale down inversely the proportion of PEG polymer incorporated into the composition as its molecular weight increases above 450-500. So, for example it is preferred not to exceed 1.5 - 2% at a molecular weight of 820, but up to 5% at 600.

According to one aspect of the present invention, there are provided anhydrous compositions containing a particulate astringent antiperspirant salt and a low molecular weight polyethylene glycol which, in the 2 day hydration test described herein, achieves a gain in hydration relative to the respective test site before treatment. Corresponding aspects include the manufacture of such compositions, in accordance with a process described herein, and the topical application to skin in accordance with a skin application described wherein.

It is advantageous in such compositions to employ polyethylene glycol which, in said 2 day hydration test, achieves a gain in hydration of at least 1.0 and preferably at least 2.5, relative to the respective test site before treatment.

The proportion of PEG can, if desired, be chosen in relation to the proportion of antiperspirant active, and in practice, a significantly or very significantly lower proportion of PEG is present compared with the astringent antiperspirant active. A convenient weight ratio of antiperspirant active to PEG polymer is selection in the range of 4:1 to 40:1 and often from 8:1 to 20:1, and particularly within such ranges when the polymer has an average weight of from 150 or 190 to 420 or 450. Such weight ratios of antiperspirant salt (AA) to PEG polymer are especially suitable in an antiperspirant formulation, such as containing at least 15% w/w and particularly at least 20% w/w of the antiperspirant salt. If a lower proportion of antiperspirant salt is used, the preferred minimum ratio AA:PEG rises from 1.5:1 at 2% AA through 2:1 at 5% AA to 3:1 at 10% AA, and for other AA proportions up to 15% by interpolation.

The benefit from blending PEG into the formulation in comparison with glycerol can be taken in either or both of two ways. In one way, an amount of PEG can be incorporated that is similar to the amount of glycerol that it would have been desired to employ, with the advantage that the agglomeration of the antiperspirant active is avoided or at least significantly reduced without the need to employ the perfume carrier. In the other way, the relative amount of PEG can be increased, without attaining the point at which undue grit is formed.

Particularly desirable compositions according to the instant invention have a grit index of not higher than 1.25, in accordance with a grit test assessed using a 4 point scale herein.

Employment of low molecular weight PEG herein exhibiting good moisturising bio-availability is particularly of benefit in conjunction with antiperspirant actives containing both aluminium and zirconium, since such actives tend to demoisturise or inhibit moisturisations to a greater extent than solely aluminium-based actives.

It is advantageous to substantially avoid incorporating an alternative uncomplexed and unbound polyhydric alcohol into the composition in order avoid at least partly eliminating the benefit obtained by employing a selected PEG polymer. By that is meant polyhydric alcohol that has not been complexed with the antiperspirant salt or bound onto the perfume carrier. The total proportion of any such unbound and uncomplexed alternative polyhydric alcohols should not exceed 1% by weight, preferably not exceed 0.5% and ideally be absent.

The carrier oil is commonly present in a weight ratio to the PEG humectant of greater than 6:1, and in many especially suitable embodiments at greater than 8:1. Said ratio is commonly less than 150:1, advantageously less than 60:1 and preferably less than 30:1. The presence of the carrier oils in a high weight ratio to the PEG reduces sensory negatives that would become apparent if a high proportion of PEG were present, such as would be the case if the PEG were used as a vehicle in which an antiperspirant active were milled.

Substantial moisturising benefit is obtainable by incorporating free low molecular weight PEG, and particularly with average molecular weight of below 620. Such formulations have been observed to address a further problem that arises from the use of certain antiperspirant formulations. The invention formulations herein can avoid or ameliorate skin irritation, or even assist in reducing axillary irritation, for example that which is shaving induced. Such a combination of benefits is especially valuable.

According to a further aspect of preferred embodiments of the present invention, there are provided anhydrous compositions containing a particulate astringent antiperspirant salt and a low molecular weight PEG humectant that reduce the irritation score in the 29 day irritation test described herein between day 0 and day 29.

It is particularly desirable to employ such compositions, optionally or preferably together with a triglyceride oil, in relative amounts of PEG and antiperspirant salt that reduce the irritation score in said 29 day irritation test between day 0 and day 29 by at least 0.5 units, and especially by at least 0.75 units.

### Gellant

The carrier oils in the invention compositions are gelled by incorporation of sufficient amount of a selected organic gellant (structurant) to attain the desired hardness of the resultant product at ambient temperature. Commonly, the gelation arises by forming a mobile liquid oil phase at an elevated temperature throughout which the gellant is distributed, and in particular by dissolution, such that when the composition cools or is cooled below its setting temperature, a firm or semi-solid product is obtained.

The proportion of gellant that it is preferred to employ depends upon a number of factors, including, in particular, the inherent capability of gellant to gel, the selection of carrier oils, the desired hardness, and finally the processing conditions such as the severity of shear that is applied during mixing in the region of the setting temperature. Accordingly, the weight of gellant is often selected in the range of from 1 part to 60 parts per 100 parts of carrier oils for firm sticks and preferably from 2 to 50 parts. The weight of gellant is often selected in the range of from 1 to 40 parts per 100 parts for a semi-solid, preferably from 2 to 30 parts.

Suitable classes of gellants include waxes, including related waxy substances, fibre-forming non-polymeric structurants, oil-soluble organic polymers, optionally copolymerised with polysilicone, and silicone elastomers.

one class of structurant which is desirable by virtue of its long standing proven capability to produce firm solids or soft or semi-solids, comprises waxes. Herein, the term wax is employed to encompass not only materials of natural origin that are solid with a waxy feel and water-insoluble, but melt or at least form a single phase with carrier oils at a somewhat higher temperature, typically between 50 and 95°C, often at least 60°C such as beeswax, candelilla spermeceti, or carnauba wax, but also other organic materials having similar properties. Such other waxes include hydrocarbon waxes, eg paraffin wax, mineral wax and microcrystalline wax, which hydrocarbon waxes may be synthetic, such as polyethylene of 400 to 10000 daltons; and waxy derivatives or waxy components of natural waxes, such as ester components identified in beeswax, be they extracted from natural beeswax, synthesised or modifications to beeswax, including such gellants as fatty alkyl (≥ C₁₆) esters, e.g. stearate esters, stearate/behenate esters, stearyl beeswax or siliconyl beeswax, for example gellants obtainable from Koster Keunen, e.g. K62, K80, K67 or K82.

Other suitable waxes include solid ester derivatives of glyceryl or glycol, typically with linear saturated fatty acids, usually containing a significant fraction of C₁₆₋₂₂ acid residues, which may be synthesised or obtained by hydrogenating the corresponding natural oil, eg the glyceride oils described hereinbefore, including castor wax. Yet others include petroleum waxes, waxy silicone polymers containing alkyl substituents of at least C₁₀ chain length; and, importantly, waxy fatty alcohols, that normally are linear and often comprise from 14 to 24 carbons, such as stearyl alcohol, cetyl alcohol or behenyl alcohol or mixtures of two or more of them, especially if obtained, maybe indirectly, from a natural feedstock.

Within the class of wax gellants, it is'often desirable to employ a mixture of gellants of differing melting/softening points, for example at least one melting at up to 70°C, such as stearyl alcohol, and another melting at above 70°C, preferably above 75°C, and particularly in the range of 75 to 90°C such as castor wax of which one commonly available wax has a melting point of about 80°C. Such combinations are particularly suitable for firm sticks. Other high melting point waxes which can be contemplated include other hydrogenated triglycerides, or aliphatic fatty esters or hydrocarbon waxes having a melting point in the desired melting point range, or blends of two or more of such waxes. These can be readily identified in literature. The weight ratio of lower to higher melting point wax for compositions herein is often in the range of from 2.5:1 to 7.5:1, and particularly from 3:1 to 6:1.

When employed as the principal or sole gellant, the total weight proportion of waxes in an invention composition herein is often selected in the range of from 6 to 25%, and in an overlapping set of compositions, expressed differently, such wax gellant constitutes advantageously from about 10 parts to about 50 parts by weight per 100 parts of carrier oils. The firmness of the resultant product increases with increasing weight proportion of gellant, especially when undue shear mixing is avoided within 5°C above down to the quiescent setting temperature of the composition.

A second class of gellants suitable for use herein comprises non-polymeric fibre-forming gellants. Such gellants, that usually are chiral, are characterised by their ability to solidify in the form of extended thin strands or fibres. Many of such fibre-forming gellants that have been identified hitherto are encompassed with the following sub-classes a) to d):-
a) hydroxystearic acid, and ester or amide derivatives thereof, including particularly 12-hydroxystearic acid, a primary gellant of USP 5650144, USP 5591424 and USP 5429816;
b) fibre-forming gellants containing amido linkage including particularly N-acyl amino acid amides and esters described in US-A-3969087, such as, in particular, N-Lauroyl-L-glutamic acid di-n-butylamide, and/or a further selection thereof in USA-2002/0159961,in which the alkyl group R³ in the N-acyl substituent -CO-R³ in its formula is characterised by containing from 7 to 10 carbon atoms, and may be branched, of which one preferred gellant comprises 2-ethyl butanoyl-L-glutamic acid di-n-butylamide; amide derivatives as set forth in WO 98/27954 notably alkyl N,N'dialkyl succinamides; cyclic ester derivatives of aspartame, namely cyclodipeptides, as set forth for example in WO 2003/059307; amido derivatives of cyclohexane as set forth in US-A-6410003;
c) Yet other fibre-forming gellants comprise lanosterol, as set forth in US-A-6251377 and a combination of a sterol and a sterol ester as set forth in WO 00/61096, eg gamma oryzanol and β-sitosterol;
d) still other fibre-forming derivatives comprise fatty acid esters of aldoses including such derivatives of maltose, as described in US6589515 and particularly fatty acid esters of cellobiose, as described for example in US6248312 and US6458344, such as in particular a product containing predominantly cellobiose octanonanoate and a minor fraction of cellobiose heptanonanoate. Yet other fibre-forming cellobiose fatty acid esters are described in WO2002/32914 in which the carboxylic acid substituent at the anomeric carbon in the cellobiose is different from the fatty acid substituent elsewhere around the cellobiose rings, for example aromatic or cycloaliphatic instead of linear alkanoate. The description herein of fibre-forming gellants includes the description of such gellants given in the respective patent specifications identified by number hereinabove.

Mixtures of materials within each sub-class of gellant/structurant a) to d) can be employed. The amount of fibre-forming gellant that is desirably employed is often selected in the range of from 2 to 30 parts per 100 parts of the oils, and especially for amido gellants, sub-class b), the weight proportion can conveniently be as low as from 2 to 10 parts per 100 parts of oil, and especially when employing an N-acyl aminoacid amide and/or a cyclodipeptide.

A further class of structurants for water-immiscible liquids that are employable herein, in accordance with their disclosure in patent literature relating to the preparation of antiperspirant formulations in soft solid or firm stick form include polymeric gellants. Examples of oil-soluble polyamides or amide/silicone copolymers are described in US6451295 or WO 9736573. The weight proportion of such polymeric oil-soluble gellants is often in the range of from 2 to 12 parts per 100 parts of oils.

The fibre-forming gellants and particularly gellants derived from aldoses, can be employed together with thickening polymers. Suitable thickening polymers include polysaccharides esterified with a fatty acid of which one excellent example comprises dextrin palmitate: polyamides as discussed in US 5500209, such as the product available under the trade name Versamid^{™} that can be derived from hexamethylene diamine and adipic acid; alkylene/arylene block copolymers, for example styrene and ethylene, propylene and/or butylene block copolymers eg those known as SEBS block copolymers, many of which are available under the trade name Kraton^{™}. The block copolymers themselves are often supplied in a compatible carrier oil, such a hydrocarbon oil. The weight proportion of such polymers (calculated as the polymer itself rather than the total of polymer plus polymer carrier) is often selected in the range of from 1 to 20 parts per 100 parts of carrier oils, the amount selected depending on the extent of thickening or structuring required, and the effectiveness of the chosen polymer in the liquid/mixture.

A further class of gellant employable herein comprises a silicone elastomer, which comprises polysiloxane strands cross-linked to a desired extent by alkylene groups. The elastomers are capable of absorbing greater, often much greater, than their own body weight of compatible oils, commonly silicone oils, hydrocarbon oils and ester oils. Suitable silicone elastomers are described for example in US5942215. The weight proportion of silicone elastomers that can be employed can desirably be the amount needed to achieve the desired product firmness by itself, often selected in the range of from 6 to 30 parts (calculated as the active itself) per 100 parts of carrier oils, or if the elastomer is employed in conjunction with an additional gellant, less than 6 parts of elastomer per 100 parts of carrier oil can be employed, such as 0.1 to 3 parts per 100 parts to benefit from the desirable sensory properties of the elastomer. The elastomer is commonly available as a gelled carrier oil, such as a volatile silicone oil, for example as described hereinbefore, typically at a concentration in the oil of from 15 to 50% by weight.

In some highly desirable embodiments of the present invention, the antiperspirant compositions comprise, in addition to the carrier oil and particulate antiperspirant active salt
i) a PEG having an average molecular weight of from 190 to 500 in an amount of 0.1 to 10% w/w,
ii) 0.1 to 6% w/w of a triglyceride oil of a C₁₆₋₁₈ unsaturated aliphatic monocarboxylic acid and
iii) 0.1 to 6% w/w of a triglyceride wax of an hydrogenated C₁₆₋₁₈ unsaturated aliphatic monocarboxylic acid.

### Optional ingredients

Optional ingredients include wash-off agents, often present in an amount of up to 5 or 10% w/w to assist in the removal of the formulation from skin or clothing. Such wash-off agents are typically nonionic surfactants such as esters or ethers containing both a C₈ to C₂₂ alkyl moiety and a hydrophilic moiety which can comprise a polyoxyalkylene group (POE or POP) and/or a polyol, eg glycerol or sorbitol.

The compositions herein can incorporate one or more cosmetic adjuncts conventionally contemplatable for cosmetic solids or soft solids. Such cosmetic adjuncts can include skin feel improvers, such as talc or finely divided high melting point polyethylene, for example in an amount of up to about 10% and often in total in an amount of from 0.5 to 6%; inorganic particulates, preferably finely divided, such as fumed silica, for example in an amount of up to 2%; skin benefit agents such as allantoin, vitamins or lipids, for example in an amount of up to 5%; colours; preservatives such as butylhydroxytoluene, often in an amount of from 0.01 to 0.1%; metal chelates, such as EDTA, for example in an amount of up to 1%; skin cooling agents, such a menthol and menthol derivatives, often in an amount of up to 2%, all of such percentages being by weight of the composition. A commonly employed adjunct is a perfume (fragrance), which is normally present at a concentration of from 0 to 4% and in many formulations from 0.25 to 2% by weight of the composition.

### Method of Manufacture

The compositions according to the present invention can be made conveniently in accordance with processes that have been employed hitherto using the same ingredients in the absence of the PEG humectant employed herein to make firm or semi solid sticks, respectively.

In general, a suitable general method of manufacture of a firm or semi-solid stick comprises the steps of
a) forming a mixture of an oil phase with an organic gellant dispersed therein;
b) heating the mixture to an elevated temperature at which the gellant becomes molten or dissolved in the oil phase;
c) introducing PEG humectant into the oil phase;
d) introducing particulate astringent antiperspirant salt into the oil phase, steps c) and d) severally being carried out before, after or simultaneously with step a or b);
e) introducing the resultant mixture containing humectant and antiperspirant salt into a dispenser, or for firm sticks alternatively into a mould and
f) cooling, or allowing said resultant mixture to cool, to below its setting temperature, at least part of this step optionally occurring before step e).

The temperature to which the dispersed mixture is heated in step b) depends on the melting or dissolution point of the chosen gellant or if a combination of gellants is employed, the one having the highest melting or dissolution temperature. This temperature is commonly at least 60°C, and in many instances is in the range of from 70 to 140°C. Preferably, the gellants and oils are selected together such that the mixture in step b) need not be heated above 100°C, and in many highly desirable embodiments is heated to a temperature of from 75 to 90°C.

In the manufacture of a semi-solid stick, it can be advantageous to subject the cooling mixture to shear mixing through its quiescent setting temperature, so as to create a cream consistency rather than a firm stick.

In the context of the above method of manufacture, organic indicates the presence of carbon in a gellant that is solid at 40°C and melts or dissolves in the oil phase at a temperature of up to 150°C.

The order of introduction of the other ingredients into the oils is at the discretion of the manufacture. In some desirable embodiments, a mixture is formed comprising oils, gellant and humectant before step b), and the astringent salt is introduced after step b), and especially after the mixture has been cooled or allowed to cool, for example to below 70°C. Post step b) introduction of the salt advantageously reduces the time for the humectant to bind salt particles before the composition attains its setting temperature, and the temperature reduction increases the viscosity of the composition to reduce the rate at which particles bump into each other.

It will be recognised that optional ingredients, if any, can be introduced at a convenient step in the process, such as hitherto employed or proposed in the absence of the humectant. Thus, any temperature sensitive ingredient is desirably introduced into the composition shortly before the dispenser is charged, and preferably at a temperature within 10°C of the setting temperature.

In an alternative method of making a soft solid composition, employing an inorganic gellant, which normally is finely particulate, the ingredients of the composition, including the oil the humectant, the antiperspirant salt and the gellant and any optional ingredient, are mixed together under high shear conditions at a convenient temperature, which may be ambient (often in the region of 20 to 25°, or at an elevated temperature, commonly up to 60 or 70°C, in order to disperse particulate constituents throughout the mixture thereafter charge the stirred mixed into a dispensing container and cool or permit the product to cool to ambient.

An alternative process that can be contemplated for making a firm stick comprises forming a mixture containing the oil, the gellant, the humectant and the antiperspirant, and subjecting the mixture to intensive mixing at a temperature below the temperature at which the gellant having the highest melting point melts, the mixing being so intense as to disperse the gellant throughout the oils and render the mixture sufficiently mobile for it to be introduced into a mould or dispenser.

Advantageously, by virtue of the properties of the PEG humectant, it can be readily incorporated into a related manufacturing process which does not employ a humectant without any detectable grit formation and without need to modify that process.

The compositions produced herein are suitable for dispensing from cosmetic dispensers for firm sticks or soft solids (semi-solids) as the case may be. Such dispensers commonly comprise a barrel, often of round or oval transverse cross section, having an opening at a first end through which the composition is dispensed and an elevator at an opposed second end that can be advanced towards the first end. The elevator fits within the barrel. Commonly, the first end can be covered with a cap, conveniently dimensioned to push it over the exterior of the barrel.

For firm sticks, the opening is the full cross section of the barrel and for soft solids the opening normally comprises a dome penetrated by a plurality of small apertures, commonly round or oval, or a slit or slits, the apertures or slits often forming a symmetrical pattern and usually constituting from about 8 to 35% of the external surface area of the dome. The elevator can be advanced by insertion of finger within the barrel or by co-operation between a threaded spindle and aperture in the elevator, the spindle being rotated by either an externally protruding rotor wheel or by a pawl arrangement. Suitable dispensers for firm sticks are described, for example in US 4232977, US4605330, W009818695, WO09603899, WO09405180, WO09325113, WO09305678, EP1040445, US5997202, US5897263, US5496122, US5275496, US 6598767, US 6299369, or WO 2002/03830. Suitable dispensers for soft or semi-solids are exemplified in US4865231, US5000356, US6116803, US5961007, WO9851185, EP0312165, WO0019860, EP0709041, EP858271, US5573341, US5725133, US5248213, US6398439 or US6450716.

The compositions of the present invention can be topically applied to skin, and particularly to underarm skin by extruding the composition in stick form above the top of the barrel or as a soft solid onto an applicator dome, as the case may be, and thereafter wiped across the skin surface, thereby depositing a fraction of the composition on the skin. The action can be repeated until the user considers that sufficient composition has been deposited, often in the region of 3 to 8 wipes per armpit. The composition is commonly applied shortly after the armpit has been washed or shaved. The composition is thereafter left in place, conventionally, for a period of time commonly between 5 and 24 hours until it is washed off, usually using soap or a conventional shower gel, and water, for example applied using a flannel, loofah, sponge or even fingers. When seeking to inhibit perspiration, the weight of antiperspirant active applied per armpit is often in the range of from 0.15 to 0.5 grams.

Particular embodiments according to the present invention are described hereinafter by way of example only. Such embodiments can be modified by the skilled person in accordance with the foregoing detailed description of the invention.

### Examples

In the Examples herein, solid or soft compositions are made using the following ingredients:-

| Ingredient | Trade name | Supplier |
|---|---|---|
| Oils | | |
| cyclomethicone | DC245 | Dow Corning |
| " | DC345 | " |
| " | DC246 | " |
| linear | DC200 (50cst) | " |
| high RI linear | DC556 | " |
| non-silicone oils | | |
| mineral oil | Hydrobrite 1000USP | Witco |
| hydrogenated polydecene | Silkflo 364 | Amoco |
| petrolatum | Vaseline | Unilever |
| ether | Fluid AP | Amerchol |
| ester | Finsolv TN | Finetex |
| isostearyl alcohol - ISA | Prisorine 3515 | Uniqema |
| emollient oils | | |
| isopropyl myristate-IPM | Estol 1514 | Uniqema |
| sunflower seed oil | Agri Pure 80 | Cargill |
| borage seed oil | borage oil | Jan Dekker |

| PEGs | | |
|---|---|---|
| PEG3 | tri(ethylene glycol) | Sigma Aldrich |
| PEG4 | Carbowax 200 | Dow Chemicals |
| PEG4³ | Polyglycol 200 | Clariant |
| PEG6 | Polyglycol 300 | Clariant |
| PEG8 | Carbowax 400 | Dow Chemicals |
| PEG8⁴ | Polyglycol 400 | Clariant |
| PEG12 | Polyglycol 600 | Clariant |
| PEG12⁵ | PEG 600 | Sigma Aldrich |
| PEG16 | Polyglycol 800 | Clariant |
| PEG20 | Polyglycol 1000 | Clariant |
| PEG32 | Polyglycol 1500 | Clariant |
| PEG40 | Polyethylene Glycol 2000 | Sigma Aldrich |
| | Polyethylene Glycol 3400 | Sigma Aldrich |
| Structurants | | |
| fatty alcohol | Lannette C18deo | Cognis |
| High MP wax, mp 80°C (92% hydrogenated caster oil) | Castorwax MP80 | CasChem |
| beeswax ester | K62 | Koster Keunen |
| silicone wax | SF1642 | General Electric |
| SMGA - cellobiose ester | CB9¹ | in house¹ |
| cyclic dipeptide | CDP² | in house ² |
| Aminoacid amide | GP1 | Ajinomoto |
| Aminoacid amide | GA-01 | " |
| Si elastomer 25% in 75% cyclomethicone | Gransil GCM | Grant |
| | | |

| Antiperspirant Actives | | |
|---|---|---|
| ACH | ACH 331 | Summit |
| AACH | Aloxicoll P | B G Giulini |
| AZAG | Reach 908 | Reheis |

| Miscellaneous | | |
|---|---|---|
| antioxidant | Ralox (BHT) | Degussa |
| fragrance | | |
| Glycerin | Pricerine 9091 | Uniqema |
| silica | Aerosil 200 | Degussa |
| talc | Suzerite 1626 | Suzerite |
| Thickener | Hydroxyethyl Cellulose | Aqualon |
| DMDMH | DMDM Hydantoin | Lonza |
| wash-off agent | Brij 700 | Uniqema |

| | | |
|---|---|---|
| CB9¹ is a cellobiose nonanoate ester made in-house in accordance with Example 1.15 of EP1199311. CDP1² is a substituted cyclic dipeptide made in-house in accordance with Example 1.2 of EP1465586. Superscripts ^{3 4} and ⁵ indicate second suppliers. | | |

### Example 1 to 8 and Comparisons C9 and C10

In these Examples, antiperspirant stick formulations were made by the following standard process. The oils, PEG, wash-off agent together with any silica, and the structurant waxes in the proportions summarised in Table 1 below were blended together and heated to approximately 85°C, by which time the wax structurants had melted to form an homogenous mixture. The mixture was permitted to cool whilst maintaining stirring until its temperature had reached about 70°C, whereupon the antiperspirant was introduced followed by the fragrance. When the mixture reached about 62/63°C, it was poured into conventional 50g dispensing canisters equipped with a platform and twist-up mechanism.

The resultant stick products were tested for grittiness by an experienced panel of evaluators in the following test procedure at laboratory ambient temperature (approximately 22°C) and assessed using a 4 point scale:-

The product is equilibrated to laboratory ambient temperature and the cap and former (if the dispenser has been bottom filled) is removed from the dispenser, to expose the stick dome above the top edge of the dispenser barrel. Visual assessment for grit is performed and then the evaluator's dry index forefinger is placed on one end of the exposed dome and wiped slowly across it (at about 0.3 - 0.4 m/s) under finger pressure to sense roughness indicating the physical presence of grit.

### Grit Scale

- 0: no observable agglomerates; smooth sensation with no perceived abrasion during finger wiping, indicating that any agglomerates were weakly bound and all disintegrated readily to impalpable particles on contact with the finger.
- 1: virtually no observable agglomerates; very minor sensation during wiping indicating that virtually all agglomerates disintegrated to impalpable particles on contact with the finger
- 2: up to a small number of observable agglomerates; minor sensation on wiping indicating that most agglomerates disintegrated to impalpable particles on contact with the finger
- 3: a significant number of agglomerates may be observed; rough sensation on application indicating that the agglomerates were firmly bound and many or all did not disintegrate to impalpable particles on contact with the finger.

At least 4 and preferably at least 12 or 16 readings are taken and averaged for each product. A product is considered not to be gritty when its averaged score is no more than 1.25.

Reference compositions for Index values 0 and 3 are made by the general process of Example 1, either free from PEG for scale 0 or substituting glycerol for PEG for scale 3, as summarised in Table 1 below.

**Table 1**

| Scale Point | 0 | 3 |
|---|---|---|
| Ingredient | % by weight | |
| DC245 | 45.5 | 42.5 |
| Fluid AP | 10 | 10 |
| Reach 908 | 24 | 24 |
| Lanette C18deo | 15 | 15 |
| Castorwax MP80 | 3.5 | 3.5 |
| Suzerite 16126 | 1 | 1 |
| Fragrance | 1 | 1 |
| Pricerine 9091 | 0 | 3 |

The results are summarised in Table 2 below.

From Table 2, it can be seen that none of the products were gritty, even when silica was absent or present in only a low weight ratio to that of the PEG humectant. This shows that silica was not needed in order to avoid grit formation. However, the products of C9 and C10 do not achieve moisturisation in the 2 day humectancy test.

**Table 2**

| Example No | 1 | 2 | 3 | 4 | 5 | 6 | 7 | 8 | C9 | C10 |
|---|---|---|---|---|---|---|---|---|---|---|
| Ingredient | Parts by weight | | | | | | | | | |
| DC245 | 32.05 | 29.70 | 32.05 | 32.70 | 29.05 | 29.70 | 32.05 | 32.05 | 32.05 | 32.05 |
| AZAG | 25.00 | 25.00 | 25.00 | 25.00 | 25.00 | 25.00 | 25.00 | 25.00 | 25.00 | 25.00 |
| Fatty Alcohol | 14.50 | 14.50 | 14.50 | 14.50 | 14.50 | 14.50 | 14.50 | 14.50 | 14.50 | 14.50 |
| Fluid AP | 10.00 | 10.00 | 10.00 | 10.00 | 10.00 | 10.00 | 10.00 | 10.00 | 10.00 | 10.00 |
| DC200 | 5.00 | 5.00 | 5.00 | 5.00 | 5.00 | 5.00 | 5.00 | 5.00 | 5.00 | 5.00 |
| Finsolv TN | 5.00 | 5.00 | 5.00 | 5.00 | 5.00 | 5.00 | 5.00 | 5.00 | 5.00 | 5.00 |
| High MP wax | 3.25 | 3.25 | 3.25 | 3.25 | 3.25 | 3.25 | 3.25 | 3.25 | 3.25 | 3.25 |
| Talc | 1.50 | 1.50 | 1.50 | 1.50 | 1.50 | 1.50 | 1.50 | 1.50 | 1.50 | 1.50 |
| Fragrance oil | 1.20 | 1.20 | 1.20 | 1.20 | 1.20 | 1.20 | 1.20 | 1.20 | 1.20 | 1.20 |
| Silica | 0.65 | - | 0.65 | 0.00 | 0.65 | - | 0.65 | 0.65 | 0.65 | 0.65 |
| Sunflower Seed Oil | 0.50 | 0.50 | 0.50 | 0.50 | 0.50 | 0.50 | 0.50 | 0.50 | 0.50 | 0.50 |
| Wash-off agent | 0.30 | 0.30 | 0.30 | 0.30 | 0.30 | 0.30 | 0.30 | 0.30 | 0.30 | 0.30 |
| Antioxidant | 0.05 | 0.05 | 0.05 | 0.05 | 0.05 | 0.05 | 0.05 | 0.05 | 0.05 | 0.05 |
| PEG4³ | 1.00 | - | - | - | - | - | - | - | - | - |
| PEG4 | - | 4.00 | - | - | - | - | - | - | - | - |
| PEG8⁴ | - | - | 1.00 | 1.00 | 4.00 | | - | - | - | - |
| PEG8 | - | - | - | - | - | 4.00 | - | - | - | - |
| PEG12 | - | - | - | - | - | - | 1.00 | - | - | - |
| PEG16 | - | - | - | - | - | - | - | 1.00 | - | - |
| PEG32 | - | - | - | - | - | - | - | - | 1.00 | - |
| PEG40 | - | - | - | - | - | - | - | - | - | 1.00 |
| Gritty Sample? | NO | NO | NO | NO | NO | NO | NO | NO | NO | NO |

### Examples 11 to 23

Further compositions in accordance with the instant invention are made by formulating products with the proportions of ingredients listed in Table 3 below. The sticks of Examples 15 to 20 are made by the standard process as for Examples 1 to 10. The sticks of Examples 11 to 14 are made by a modified process in which the amido or peptide structurant(s) is/are dissolved in a preliminary stage in the ISA by heating the mixture with stirring to around 90°C. The remaining oils and any remaining structurant are mixed and heated to about 85°C. The two fractions are combined and the resulting mixture is subsequently treated in the same way as in the standard process.

Examples 21 and 22 are made by a variation to the standard process in which the mixture containing all the ingredients that has been formed at an elevated temperature is cooled and subjected to high shear mixing during the cooling process until and through the quiescent setting temperature, so that the final product is in the form of a soft solid. The dispenser into which these products are poured further comprises a convex dome applicator head having a plurality of slits through which the composition can be extruded by elevation of the platform. The product of Example 23 is made by mixing all the ingredients at ambient temperature, heating the mixture to about 50°C and subjecting it to high shear mixing, and thereafter pouring the mixture into the soft-solid dispenser.

**Table 3**

| | Firm Sticks | | | | | | | | | | Soft Solids | | |
|---|---|---|---|---|---|---|---|---|---|---|---|---|---|
| Ingredients | parts by weight | | | | | | | | | | | | |
| Example No | 11 | 12 | 13 | 14 | 15 | 16 | 17 | 18 | 19 | 20 | 21 | 22 | 23 |
| DC245 | | 20 | 7.7 | 12 | | 37 | | | | 36 | 44.3 | 33 | |
| DC345 | 33 | | | | 30 | | 35 | | 20 | | | | 35 |
| DC246 | | | | | | | | 34.5 | | | | | |
| DC200 | 5 | | 10 | | | | | | 5 | 12 | | 7.7 | |
| DC556 | | 10 | | | 15 | | | | | | 10 | | 5 |
| Mineral Oil | | | | | | | | 4 | | | | | 6 |
| Silkflo 364 | 5 | | | | | 15 | | | | | 6 | | |
| Petrolatum | | | 15 | 15 | | | | | | | | | |
| Fluid AP | | 12 | 7.7 | 12 | 15 | | 15 | | | | | | 5.5 |
| Finsolv TN | 7 | | 7.7 | | | | | | 8 | | | 12 | |
| ISA | 20 | 24 | 27.3 | 22.2 | | | | | | 10 | | | |
| IPM | 5 | | | | | 2 | 3 | 4 | | | | 6 | 5 |
| SSO | | | 2 | | 2 | 2 | | | | | 1.2 | | 2.7 |
| Borage Oil | | 1.8 | | | | | | | 1 | 1.5 | 1 | 1.2 | |
| PEG3 | | | | | 2 | | | | 0.5 | | | | |
| PEG4 | | | | | | | | 2 | | 0.5 | | 4 | |
| PEG6 | | 3 | | | | 4 | | | | | | | 2 |
| PEG8 | | | | | | | | | 2 | 4 | | | 2 |
| PEG12 | | 1 | 6 | 6 | | | | | | | 1 | | |
| PEG16 | | | | | | | 3 | | | | | | |
| Fatty Alcohol | | | | | | | 14 | 14.5 | | 13 | | 4 | |
| High MP wax | | 1 | | | | 2 | 4 | 3.5 | | 4 | 5 | | |
| K62 | | | | | | 12 | | | 14 | | | | |
| SF1462 | | | | | | | | | | 2 | 3 | | |
| CB9¹ | | | | | 10 | | | | | | | | |
| CDP1² | 4.5 | | | | | | | | | | | | |
| GP1 | | 2.5 | 4 | 3.25 | | | | | | | | | |
| GA-01 | | 3.5 | 4 | 3.25 | | | | | | | | | |
| Si Elastomer | | | | | | | | | 20 | | | 12 | |
| ACH | | | | | 25 | 22 | | 24 | | | | 18 | |
| AACH | | 20 | | | | | | | 25 | 21 | 20 | | |
| AZAG | 15 | | 25 | 25 | | | 23 | | | | | | 24 |
| antioxidant | | | 0.1 | 0.1 | | 0.1 | | 0.05 | | | | 0.1 | |
| fragrance | | 1.2 | 1.2 | 1.2 | | | 1 | | 1.5 | 0.8 | | 2 | |
| silica | 0.5 | | | | 0.3 | | 0.7 | | | | | | 6 |
| talc | 6 | | | | | 3 | | | | | 8 | | 6 |
| wash-off agent | 0.5 | | | | 0.5 | 0.9 | 1.5 | 1.45 | 1.2 | | 0.5 | | 0.8 |

### Example 24 and Comparison C25

In this Example, a skin benefit of employing a composition employing a PEG is demonstrated, by comparing the elastic constant of skin when treated under the same conditions with a composition containing a PEG and the same composition in which the PEG was replaced by the same proportion of the volatile silicone carrier oil, compositions being summarised in Table 4.

Over a period of 5 days, 0.2g samples of the invention and reference compositions were applied twice daily (once only on the fifth day) to separate 20cm² sites on volar forearm skin on the same arm of 16 subjects, which had been lightly shaved before the test commenced. Shortly before the second application on days 1 to 4 and 5 hours after the single application on day 5, the elastic constant of the skin was measured by a Dermal Torque Meter ™ from Dia-Stron Ltd, Andover, UK, and the results summarised in Table 5 below.

In addition, the trans epidermal water loss was measured daily using a Dermalab Evaporimeter from Cortex Technologies. The measurement on Day 5 is compared with the measurement on Day 1 to determine whether there had been any change in water loss through the epidermis, and the average for the subjects is summarised in Table 6 below.

The compositions were as follows:-

**Table 4**

| | C25 | Ex 24 |
|---|---|---|
| Ingredients | % by weight | |
| DC-245 | 33.24 | 29.24 |
| AZAG Tetra | 25.0 | 25.0 |
| Fatty alcohol | 14.50 | 14.50 |
| Fluid AP | 10.00 | 10.00 |
| DC-200 (50 CST) | 5.00 | 5.00 |
| Finsolv TN | 5.00 | 5.00 |
| PEG8 | | 4.00 |
| High MP Wax | 3.25 | 3.25 |
| Talc | 1.50 | 1.50 |
| Fragrance Oil | 1.00 | 1.00 |
| Silica | 0.65 | 0.65 |
| Sunflower Seed Oil | 0.50 | 0.50 |
| Wash-off agent | 0.30 | 0.30 |
| antioxidant | 0.05 | 0.05 |
| Glycerin | 0.01 | 0.01 |

**Table 5**

| Day | Average Dermal Torque Reading | |
|---|---|---|
| | Example 24 | PEG-free C25 |
| 1 | 0 | 0 |
| 2 | -1.7 | -6.25 |
| 3 | -4.7 | -8.1 |
| 4 | -4.4 | -8.6 |
| 5 | -3.7 | -8.3 |

From Table 5 above, it can be seen that the skin that was contacted with the antiperspirant composition containing the separately introduced PEG humectant demonstrated consistently a higher reading for Elastic Constant than did the skin which had been treated in exactly the same way with the PEG-free composition. This shows that the PEG had improved the flexibility of the skin and had at least partly counter-acted the negative effect on stratum corneum from applying the antiperspirant composition.

**Table 6**

| | PEG free C25 | Example 24 |
|---|---|---|
| Trans Epidermal Water Loss TEWL) (g/M²/hr) | + 0.27 | -0.73 |

Table shows that the TEWL was better using the invention product than using the comparison product which did not contain any PEG8, and indeed the +ve change is better than no change, but even shows a reduction in the rate of water loss. This shows that the condition of the epidermis had been improved by use of the invention product, containing the separately introduced PEG humectant, even though it also contained about 25% by weight of an astringent antiperspirant salt.

### 29 day irritation test

In a further trial, the skin benefit of the formulation of Example 24 was carried out to show that it reduced the irritation perceived by users that had previously applied a reference stick containing AZAG but no PEG.

The 29 day irritation test is conducted on 15 female panellists aged between 18 and 55 for each product or control tested. Two products or product plus control are usually tested simultaneously, involving 30 panellists.

The test procedure lasts 5 weeks, commencing on a Monday, of which the first week represents a provocation phase and the subsequent four weeks a recovery phase. Throughout the test panellists are instructed to shave their underarms on each Wednesday and Saturday evenings, solely using disposable razors after application of a wetted mild soap bar and thereafter rinsing with water.

In the provocation phase, the panellists apply four times daily under both arms an antiperspirant stick having the following composition:-

**Table 7**

| Ingredient | % by weight |
|---|---|
| 12-hydroxy stearic acid | 7.00 |
| N-lauroyl glutamic acid | 2.00 |
| di-n-butyl amide | |
| Cyclomethicone DC245 | 46.90 |
| Octyl dodecanol | 14.00 |
| AZAG Reach 908 | 26.0 |
| C₂₀₋₄₀ Pareth 40 | 2.50 |
| C₂₀₋₄₀ Alcohol | 0.50 |
| NA EDTA | 0.10 |
| Fragrance | 1.00 |

During the subsequent recovery phase, each panellist applies one product four times daily under the left arm and a second product four times daily under the right arm, the allocation being randomised between panellists to achieve a balance of left and right arm application for the test and second test or control products. The sticks are weighed weekly to confirm that panellists are applying consistent amounts to each arm. The skin condition of the panellists is assessed on Monday, Wednesday and Friday of each week. The measurement on Day 0 (the baseline value) is made on the first day (Monday) of the recovery phase immediately before the first product application. Day 29 is the Monday, 4 weeks later.

The irritation suffered by the panellists is assessed by a trained analyst against a 5 point scale, ranging from 0 to 4 in which the assessment criteria are as follows (chosen criterion depends on which signs are detected):-

| **Value** | **Description** |
|---|---|
| 0.0 | No apparent cutaneous involvement.; |
| | No Folliculitis (F) or Urticaria |
| 0.5 | Faint, barely perceptible erythema ; slight dryness; |
| | Just visible Folliculitis (F) or Urticaria |
| 1.0 | Faint but definite erythema, no eruptions or broken skin; or no erythema but definite dryness, may have epidermal fissuring; |
| | Slight reaction - Folliculitis (F) or Urticaria |

| **Value** | **Description** |
|---|---|
| 1.5 | Well defined erythema or faint erythema with definite dryness, may have epidermal fissuring; |
| | Moderate Folliculitis (F) or Urticaria. |
| 2.0 | Moderate erythema, may have very few papules deep fissures, or moderate to severe erythema in the cracks.; |
| | Distinct Folliculitis (F) or Urticaria |
| 2.5 | Moderate erythema with barely perceptible oedema or severe erythema not involving a significant portion of the patch (halo effect around the edges), may have a few papules or moderate to severe erythema. |
| | Well developed Folliculitis (F) or Urticaria |
| 3.0 | Severe erythema (beet redness), may have generalized papules or moderate to severe erythema with slight oedema (edges well defined by raising) ; |
| | Strong Folliculitis (F) or Urticaria. |
| 3.5 | Moderate to severe erythema with moderate oedema (confined to patch area) or moderate to severe erythema with isolated eschar formations or vesicles. |
| | Very strong Folliculitis (F) or Urticaria |
| 4.0 | Generalized vesicles or eschar formations or moderate to severe erythema and/or oedema extending beyond the area of the patch; |
| | Extremely strong Folliculitis (F) or Urticaria |

The assessed scores for the composition of Example 24 were averaged and are given weekly in Table 8 below.

**Table 8**

| Assessment (Days into Recovery Phase) | Example 24 |
|---|---|
| 0 | 1.68 |
| 8 | 1.20 |
| 15 | 0.96 |
| 22 | 0.86 |
| 29 | 0.60 |

From Table 8, it can be seen that the Example product increasingly reduced irritation relative to the provocation product, showing that the stick containing PEG-8 is very effective at avoiding and reducing the irritation arising from shaving and like actions commonly carried out in the axilla, which continued to be carried out during the recovery phase.

### Examples 26, 27 and Comparisons C28, C29

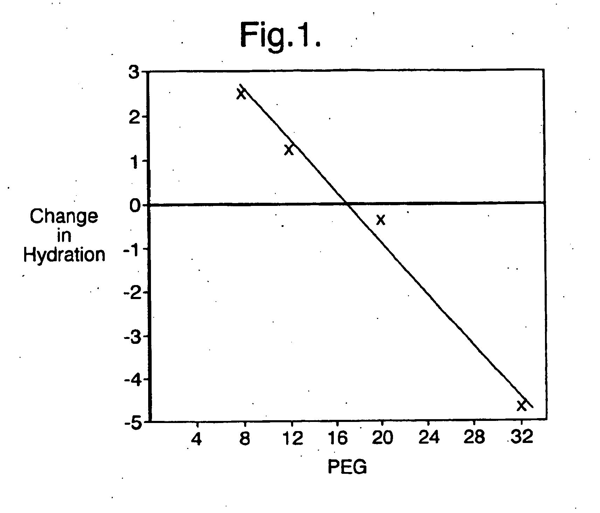

These Examples and Comparisons demonstrate the comparative capability of PEG polymers of differing average molecular weight to moisturise skin in a 2 day hydration test. The materials tested and the resultant change in hydration is summarised in Table 9 and the results plotted in Figure 1.

The 2 day hydration test is conducted as follows, using at least 16 sites per material to be tested, subjects being aged between 18 and 55. The test employs a 4% by weight solution of the potential humectant in distilled water containing 0.5% by weight DMDMH and thickened with 1% by weight hydroxyethylcellulose.

In the test, both volar forearms of each subject are employed, 3 sites per forearm. In each set of trials, baseline measurements of skin hydration are made for each site, followed by immediate application of test products randomised across the various test sites of the subjects. A second application of the same product is made 5 hours later and a third application made after a further 19 hours. Skin hydration of each site is measured after a further 5 hours at the end of the test, i.e. 29 hours after the first application. The difference between the initial and final measurements for each set are averaged

Hydration is measured using a Corneometer CM825 obtainable from Courage and Khazaka. Each test site is a 3x3cm square, to which is applied 0.05g of test product in each application. The measurements are made under temperature and humidity controlled conditions, 20°C and 50%RH, in which the subjects had been conditioned for 15 minutes before the hydration measurements were made. Subjects were instructed to avoid consuming caffeine for 30 minutes before the skin hydration measurements were made and to avoid washing or otherwise submersing their forearms in water, and not to apply moisturising creams or lotions to the forearm during the 29 hours of the trial.

**Table 9**

| | Ex 26 | Ex 27 | C28 | C29 |
|---|---|---|---|---|
| Ingredients | % by weight | | | |
| PEG8⁴ | 4 | 0 | 0 | 0 |
| PEG12 | 0 | 4 | 0 | 0 |
| PEG20 | 0 | 0 | 4 | 0 |
| PEG32 | 0 | 0 | 0 | 4 |
| Thickener | 1 | 1 | 1 | 1 |
| DMDMH | 0.5 | 0.5 | 0.5 | 0.5 |
| Distilled Water | 94.5 | 94.5 | 94.5 | 94.5 |
| Change in Hydration | 2.5 | 1.2 | -0.4 | -4.7 |

From Table 9 and as graphically illustrated in Figure 1, the moisturising capability of PEG progressively diminished as its molecular weight increased from PEG-8 (about 400) in Example 26 upwards. In comparison C29, there was a small dehydration compared with the initial measurement and by Comparison 29, there was a much larger demoisturisation. By interpolation, positive hydration was achievable at a PEG molecular weight of about 820 and lower.

## Claims

1. An anhydrous antiperspirant or deodorant composition comprising
a particulate astringent antiperspirant salt;
20 to 90% by weight of a carrier oil,
a gellant for the carrier oil and
a polyol humectant,
in which the polyol humectant comprises 0.1 to 10% by weight of the composition polyethylene glycol having an average molecular weight of up to 820.

2. A composition according to claim 1 in which the polyethylene glycol has an average molecular weight of from 150 to 620.

3. A composition according to claim 2 in which the polyethylene glycol has an average molecular weight of at least 190.

4. A composition according to claim 2 or 3 in which the polyethylene glycol has an average molecular weight of up to 520.

5. A composition according to any preceding claim containing at least 0.5% by weight of the polyethylene glycol.

6. A composition according to any preceding claim containing up to 7.5% by weight of the polyethylene glycol.

7. A composition according to claim 6 containing 0.5 to 5% by weight of the polyethylene glycol.

8. A composition according to any preceding claim in which the particulate astringent antiperspirant salt and the polyethylene glycol are present in a weight ratio of from 4:1 to 40:1.

9. A composition according to claim 8 in which the particulate astringent antiperspirant salt and the polyethylene glycol are present in a weight ratio of up to 20:1.

10. A composition according to any of claims 5 to 9 in which the polyethylene glycol has an average molecular weight of from 150 to 500.

11. A composition according to any preceding claim in which the astringent antiperspirant salt comprises a chlorohydrate salt of aluminium and/or zirconium, optionally complexed.

12. A composition according to claim 11 in which the astringent antiperspirant salt is an activated aluminium chlorohydrate.

13. A composition according to claim 11 in which the astringent antiperspirant salt is an aluminium-zirconium chlorohydrate, optionally activated and/or complexed with an amino acid.

14. A composition according to claim 11 in which at least 99% by weight of the astringent antiperspirant salt has a particle diameter of below 50 microns.

15. A composition according to any preceding claim which contains from 40 to 90% by weight of the carrier oil.

16. A composition according to claim 15 which contains from 45 to 60% by weight of the carrier oil.

17. A composition according to any preceding claim in which the carrier oil includes a volatile silicone oil.

18. A composition according to claim 17 in which the volatile silicone oil comprises cyclomethicone.

19. A composition according to claim 18 in which the volatile silicone oil constitutes at least 20% by weight of the carrier oil.

20. A composition according to any preceding claim in which the carrier oil comprises an ester oil.

21. A composition according to claim 20 in which the ester oil comprises an aromatic ester oil.

22. A composition according to claim 20 or 21 in which the ester oil constitutes at least 10% by weight of the carrier oil.

23. A composition according to any preceding claim in which the carrier oil comprises a non-volatile ether oil.

24. A composition according to claim 23 in which the ether oil constitutes up to 30% by weight of the carrier oil.

25. A composition according to any preceding claim in which the carrier oil comprises an ester oil and an ether oil in a weight ratio of from 5:1 to 1:5.

26. A composition according to claim 23, 24 or 25 wherein the non-volatile ether oil comprises an alkyl ether of polypropylene glycol.

27. A composition according to any preceding claim which includes a natural triglyceride oil.

28. A composition according to any preceding claim which contains from 2 to 25 parts by weight.gellant per 100 parts of carrier oil.

29. A composition according to any preceding claim in which the gellant comprises a wax.

30. A composition according to claim 29 in which the wax gellant comprises a linear fatty alcohol.

31. A composition according to any preceding claim in which the gellant comprises a mixture of a linear fatty alcohol and a wax melting at a temperature of at least 75°C.

32. A composition according to claim 30 or 31 in which the linear fatty alcohol comprises stearyl alcohol.

33. A composition according to any of claims 1 to 28 which is gelled with a non-polymeric fibre-forming gellant.

34. A composition according to claim 33 in which the gellant comprises an N-acylamino acid amide.

35. A composition according to claim 28 in which the gellant is employed in weight ratio of from 2 to 10 parts per 100 parts by weight of carrier oil.

36. A composition according to any preceding claim in which the carrier oil is present in a weight ratio to the polyethylene glycol of at least 6:1.

37. A composition according to any preceding claim in which the carrier oil is present in a weight ratio to the polyethylene glycol of less than 30:1

38. A product comprising a composition according to any preceding claim disposed with a dispenser comprising a barrel having at one end at least one opening through which the composition can be expelled and an opposite second end comprising an elevator fitting within the barrel and capable of being advanced towards the first end.

39. A non-therapeutic method of inhibiting perspiration or body odour comprising the step of topically applying to skin a composition according to any of claims 1 to 37.

## Patentansprüche

1. Wasserfreie transpirationshemmende oder desodorierende Zusammensetzung, umfassend
ein partikuläres, adstringentes, transpirationshemmendes Salz;
20 bis 90 Gewichts-% eines Trägeröls,
ein Geliermittel für das Trägeröl und
ein Polyol-Feuchthaltemittel,
wobei das Polyol-Feuchthaltemittel 0,1 bis 10 Gewichts-%, bezogen auf die Zusammensetzung, Polyethylenglykol, das ein durchschnittliches Molekulargewicht von bis zu 820 hat, umfasst.

2. Zusammensetzung gemäß Anspruch 1, wobei das Polyethylenglykol ein durchschnittliches Molekulargewicht von 150 bis 620 hat.

3. Zusammensetzung gemäß Anspruch 2, wobei das Polyethylenglykol ein durchschnittliches Molekulargewicht von wenigstens 190 hat.

4. Zusammensetzung gemäß Anspruch 2 oder 3, wobei das Polyethylenglykol ein durchschnittliches Molekulargewicht von bis zu 520 hat.

5. Zusammensetzung gemäß einem vorangehenden Anspruch, die wenigstens 0,5 Gewichts-% des Polyethylenglykols enthält.

6. Zusammensetzung gemäß einem vorangehenden Anspruch, die bis zu 7,5 Gewichts-% des Polyethylenglykols enthält.

7. Zusammensetzung gemäß Anspruch 6, die 0,5 bis 5 Gewichts-% des Polyethylenglykols enthält.

8. Zusammensetzung gemäß einem vorangehenden Anspruch, wobei das partikuläre, adstringente, transpirationshemmende Salz und das Polyethylenglykol in einem Gewichtsverhältnis von 4:1 bis 40:1 vorliegen.

9. Zusammensetzung gemäß Anspruch 8, wobei das partikuläre, adstringente, transpirationshemmende Salz und das Polyethylenglykol in einem Gewichtsverhältnis von bis zu 20:1 vorliegen.

10. Zusammensetzung gemäß einem der Ansprüche 5 bis 9, wobei das Polyethylenglykol ein durchschnittliches Molekulargewicht von 150 bis 500 hat.

11. Zusammensetzung gemäß einem vorangehenden Anspruch, wobei das adstringente, transpirationshemmende Salz ein Chlorhydratsalz von Aluminium und/oder Zirkonium, gegebenenfalls komplexiert, umfasst.

12. Zusammensetzung gemäß Anspruch 11, wobei das adstringente, transpirationshemmende Salz ein aktiviertes Aluminiumchlorhydrat umfasst.

13. Zusammensetzung gemäß Anspruch 11, wobei das adstringente, transpirationshemmende Salz ein Aluminium-Zirkonium-Chlorhydrat, gegebenenfalls aktiviert und/oder komplexiert mit einer Aminosäure, ist.

14. Zusammensetzung gemäß Anspruch 11, wobei wenigstens 99 Gewichts-% des adstringenten, transpirationshemmenden Salzes einen Partikeldurchmesser von unter 50 Mikrometer haben.

15. Zusammensetzung gemäß einem vorangehenden Anspruch, die 40 bis 90 Gewichts-% des Trägeröls enthält.

16. Zusammensetzung gemäß Anspruch 15, die 45 bis 60 Gewichts-% des Trägeröls enthält.

17. Zusammensetzung gemäß einem vorangehenden Anspruch, wobei das Trägeröl ein flüchtiges Silikonöl umfasst.

18. Zusammensetzung gemäß Anspruch 17, wobei das flüchtige Silikonöl Cyclomethicon umfasst.

19. Zusammensetzung gemäß Anspruch 18, wobei das flüchtige Silikonöl wenigstens 20 Gewichts-% des Trägeröls bildet.

20. Zusammensetzung gemäß einem vorangehenden Anspruch, wobei das Trägeröl ein Esteröl umfasst.

21. Zusammensetzung gemäß Anspruch 20, wobei das Esteröl ein aromatisches Esteröl umfasst.

22. Zusammensetzung gemäß Anspruch 20 oder 21, wobei das Esteröl wenigstens 10 Gewichts-% des Trägeröls bildet.

23. Zusammensetzung gemäß einem vorangehenden Anspruch, wobei das Trägeröl ein nicht-flüchtiges Etheröl umfasst.

24. Zusammensetzung gemäß Anspruch 23, wobei das Etheröl bis zu 30 Gewichts-% des Trägeröls bildet.

25. Zusammensetzung gemäß einem vorangehenden Anspruch, wobei das Trägeröl ein Esteröl und ein Etheröl in einem Gewichtsverhältnis von 5:1 bis 1:5 umfasst.

26. Zusammensetzung gemäß Anspruch 23, 24 oder 25, wobei das nicht-flüchtige Etheröl einen Alkylether von Polypropylenglykol umfasst.

27. Zusammensetzung gemäß einem vorangehenden Anspruch, die ein natürliches Triglyceridöl umfasst.

28. Zusammensetzung gemäß einem vorangehenden Anspruch, die 2 bis 25 Gewichtsteile Geliermittel pro 100 Teile Trägeröl enthält.

29. Zusammensetzung gemäß einem vorangehenden Anspruch, wobei das Geliermittel ein Wachs umfasst.

30. Zusammensetzung gemäß Anspruch 29, wobei das Wachs-Geliermittel einen linearen Fettalkohol umfasst.

31. Zusammensetzung gemäß einem vorangehenden Anspruch, wobei das Geliermittel ein Gemisch aus einem linearen Fettalkohol und einem Wachs, das bei einer Temperatur von wenigstens 75 °C schmilzt, umfasst.

32. Zusammensetzung gemäß Anspruch 30 oder 31, wobei der lineare Fettalkohol Stearylalkohol umfasst.

33. Zusammensetzung gemäß einem der Ansprüche 1 bis 28, die mit einem nicht-polymeren Faser-bildenden Geliermittel geliert ist.

34. Zusammensetzung gemäß Anspruch 33, wobei das Geliermittel ein N-Acylaminosäureamid umfasst.

35. Zusammensetzung gemäß Anspruch 28, wobei das Geliermittel in einem Gewichtsverhältnis von 2 bis 10 Teilen pro 100 Gewichtsteile Trägeröl verwendet wird.

36. Zusammensetzung gemäß einem vorangehenden Anspruch, wobei das Trägeröl in einem Gewichtsverhältnis zu dem Polyethylenglykol von wenigstens 6:1 vorliegt.

37. Zusammensetzung gemäß einem vorangehenden Anspruch, wobei das Trägeröl in einem Gewichtsverhältnis zu dem Polyethylenglykol von weniger als 30:1 vorliegt.

38. Produkt, umfassend eine Zusammensetzung gemäß einem vorangehenden Anspruch, ausgestattet mit einem Spender, der einen Zylinder umfasst, der an einem Ende wenigstens eine Öffnung hat, durch welche die Zusammensetzung ausgestoßen werden kann, und ein gegenüberliegendes zweites Ende hat, das ein Hebeverbundstück innerhalb des Zylinder umfasst und fähig ist, in Richtung des ersten Endes vorwärts bewegt zu werden.

39. Nicht-therapeutisches Verfahren zur Hemmung der Schweißbildung oder von Körpergeruch, umfassend den Schritt eines topischen Auftragens einer Zusammensetzung gemäß einem der Ansprüche 1 bis 37 auf Haut.

## Revendications

1. Composition antitranspirante ou déodorante anhydre comprenant
◆ un sel antitranspirant astringent particulaire,
◆ 20 à 90% en poids d'un véhicule huileux,
◆ un gélifiant pour le véhicule huileux et
◆ un humectant de type polyol,
dans laquelle l'humectant de type polyol comprend 0,1 à 10%, en poids de la composition, de polyéthylèneglycol ayant une masse moléculaire moyenne allant jusqu'à 820.

2. Composition selon la revendication 1 dans laquelle le polyéthylèneglycol a une masse moléculaire moyenne de 150 à 620.

3. Composition selon la revendication 2 dans laquelle le polyéthylèneglycol a une masse moléculaire moyenne d'au moins 190.

4. Composition selon la revendication 2 ou 3 dans laquelle le polyéthylèneglycol a une masse moléculaire moyenne allant jusqu'à 520.

5. Composition selon l'une quelconque des revendications précédentes contenant au moins 0,5% en poids du polyéthylèneglycol.

6. Composition selon l'une quelconque des revendications précédentes contenant jusqu'à 7,5% en poids du polyéthylèneglycol.

7. Composition selon la revendication 6 contenant 0,5 à 5% en poids du polyéthylèneglycol.

8. Composition selon l'une quelconque des revendications précédentes dans laquelle le sel antitranspirant astringent particulaire et le polyéthylèneglycol sont présents dans un rapport pondéral de 4:1 à 40:1.

9. Composition selon la revendication 8 dans laquelle le sel antitranspirant astringent particulaire et le polyéthylèneglycol sont présents dans un rapport pondéral allant jusqu'à 20:1.

10. Composition selon l'une quelconque des revendications 5 à 9 dans laquelle le polyéthylèneglycol a une masse moléculaire moyenne de 150 à 500.

11. Composition selon l'une quelconque des revendications précédentes dans laquelle le sel antitranspirant astringent comprend un sel chlorhydrate d'aluminium et/ou de zirconium, éventuellement complexé.

12. Composition selon la revendication 11 dans laquelle le sel antitranspirant astringent est un chlorhydrate d'aluminium activé.

13. Composition selon la revendication 11 dans laquelle le sel antitranspirant astringent est un chlorhydrate d'aluminium-zirconium, éventuellement activé et/ou complexé avec un acide aminé.

14. Composition selon la revendication 11 dans laquelle au moins 99% en poids du sel antitranspirant astringent a un diamètre particulaire qui est inférieur à 50 microns.

15. Composition selon l'une quelconque des revendications précédentes qui contient de 40 à 90% en poids du véhicule huileux.

16. Composition selon la revendication 15 qui contient de 45 à 60% en poids du véhicule huileux.

17. Composition selon l'une quelconque des revendications précédentes dans laquelle le véhicule huileux contient une huile de silicone volatile.

18. Composition selon la revendication 17 dans laquelle l'huile de silicone volatile comprend de la cyclométhicone.

19. Composition selon la revendication 18 dans laquelle l'huile de silicone volatile constitue au moins 20% en poids du véhicule huileux.

20. Composition selon l'une quelconque des revendications précédentes dans laquelle le véhicule huileux comprend une huile d'ester.

21. Composition selon la revendication 20 dans laquelle l'huile d'ester comprend une huile d'ester aromatique.

22. Composition selon la revendication 20 ou 21 dans laquelle l'huile d'ester constitue au moins 10% en poids du véhicule huileux.

23. Composition selon l'une quelconque des revendications précédentes dans laquelle le véhicule huileux comprend une huile d'éther non volatile.

24. Composition selon la revendication 23 dans laquelle l'huile d'éther constitue jusqu'à 30% en poids du véhicule huileux.

25. Composition selon l'une quelconque des revendications précédentes dans laquelle le véhicule huileux comprend une huile d'ester et une huile d'éther dans un rapport pondéral de 5:1 à 1:5.

26. Composition selon la revendication 23, 24 ou 25 dans laquelle l'huile d'éther non volatile comprend un éther alkylique de polypropylène-glycol.

27. Composition selon l'une quelconque des revendications précédentes qui contient une huile triglycéride naturelle.

28. Composition selon l'une quelconque des revendications précédentes qui contient de 2 à 25 parties en poids de gélifiant pour 100 parties de véhicule huileux.

29. Composition selon l'une quelconque des revendications précédentes dans laquelle le gélifiant comprend une cire.

30. Composition selon la revendication 29 dans laquelle le gélifiant cireux comprend un alcool gras linéaire.

31. Composition selon l'une quelconque des revendications précédentes dans laquelle le gélifiant comprend un mélange d'un alcool gras linéaire et d'une cire fondant à une température d'au moins 75°C.

32. Composition selon la revendication 30 ou 31 dans laquelle l'alcool gras linéaire comprend de l'alcool stéarylique.

33. Composition selon l'une quelconque des revendications 1 à 28 qui est gélifiée avec un gélifiant formant des fibres non polymères.

34. Composition selon la revendication 33 dans laquelle le gélifiant comprend un amide de N-acylaminoacide.

35. Composition selon la revendication 28 dans laquelle le gélifiant est employé dans un rapport pondéral de 2 à 10 parties pour 100 parties en poids de véhicule huileux.

36. Composition selon l'une quelconque des revendications précédentes dans laquelle le véhicule huileux est présent dans un rapport pondéral par rapport au polyéthylèneglycol d'au moins 6:1.

37. Composition selon l'une quelconque des revendications précédentes dans laquelle le véhicule huileux est présent dans un rapport pondéral par rapport au polyéthylèneglycol inférieur à 30:1.

38. Produit comprenant une composition selon l'une quelconque des revendications précédentes dotée d'un distributeur comprenant un corps comportant, à l'une de ses extrémités, au moins une ouverture par laquelle la composition peut être expulsée et une seconde extrémité opposée comprenant un élévateur situé à l'intérieur du corps et susceptible d'être déplacé vers la première extrémité.

39. Procédé non thérapeutique permettant d'inhiber la transpiration ou les odeurs corporelles comprenant l'étape consistant à appliquer localement sur la peau une composition selon l'une quelconque des revendications 1 à 37.
